# EUROPEAN PATENT SPECIFICATION

(11) **EP 3 313 321 B1**
(45) Date of publication and mention of the grant of the patent: **02.10.2024**
(21) Application number: 16731182.8
(22) Date of filing: 23.06.2016
(51) Int. Cl.: A61C 17/06

(54) **DENTAL SUCTION ARRANGEMENT**
DENTALES SAUGINSTRUMENT
INSTRUMENT D'ASPIRATION DENTAIRE

(30) Priority: 26.06.2015 SE 1550893
(43) Date of publication of application: 02.05.2018
(73) Proprietor: Asum, Thomas, 430 85 Brännö (SE)
(72) Inventor: Asum, Thomas, 430 85 Brännö (SE)
(74) Representative: Baldus, Oliver
(86) International application number: PCT/EP2016/064618
(87) International publication number: WO 2016/207317

(56) References cited:
- GB-A- 1 060 288
- US-A- 1 742 080
- US-A- 5 071 347
- US-A1- 2013 203 012
- ANONYMOUS: "J.H. Orsing AB", 18 February 2015 (2015-02-18), XP055801872, Retrieved from the Internet <URL:https://web.archive.org/web/20150218042400/http://orsing.se/products/saliva-ejectors/hygoformic/> [retrieved on 20210506]

## Description

### Technical field

The invention is about a dental suction arrangement which removes saliva and other fluids from a person's oral cavity. The suction arrangement includes a suction tube and an absorption body to be placed in the oral cavity, thereby the suction tube includes a suction part which is arranged in the absorption body and which is equipped with a hole member communicating with the absorption body.

### Technical background

in the dental field different types of suction arrangements are used for removal of saliva and water from the oral cavity. The supply of water mainly takes place when the tooth substance is drilled and grinded and when removing tartar; o cool the teeth - using water spray - is very pleasant to the user, in order to lower te mouth temperature. Water supply is also common in connection with teeth - and oral cavity - cleaning where water spray is used. The removal of saliva and water is beneficial for several reasons, including patient's or user's comfort, working area visibility, dry keeping of an operating area, The removal of saliva and water is required for several reasons for example user's comfort, to improve visibility in the cleaning process, and to keep the operating area dry.

The removal of saliva and water is mainly performed using two kinds of suction devices including tubes with different dimensions, where the thicker one is a so called high-speed suction device that efficiently removes large volumes of water and saliva, and which is operated manually by the operator or his assistant. The suction device with a thinner dimension and lower suction capacity is mainly used in connection with a device which is fixed in the oral cavity and where the removal of saliva and water works automatically without impact of the operator. Often the suction device also works as a protection of the tongue against rotating instruments by keeping away the tongue. One example of such a thinner dimensioned suction device is described in US 3,086,289. These kinds of suction arrangements consist solely of various kinds of plastic tubes, straight or pre-bent in different shapes, with holes placed in one end or along the tube. By reshaping the suction device by hand it is possibly to adjust the device to different locations in the oral cavity where the absorption at the moment it is most needed and where the suction device brings most benefit.

The problem is that all people look different in combination with the fact that the constructions are rather static with limited possibilities for adjustments of the absorption effect to areas where it is most needed. These constructions are neither able to absorb salvia and water further down at the bottom of the mouth adjacent to the Glandula Sublingualis nor further back in the throat and they have only limited ability to safe isolate an operating area from saliva since the absorption effect is limited to the area where the suction device's holes are located.

Another major problem is the low comfort of these constructions. The need to remove saliva and water is often highest at the bottom of the mouth but when the suction device is placed there it almost always will be contact with the inside of the lower jaw, which is sensitive to pain and it often results in a strong discomfort for the patient or user. To minimize this effect, now and then a need arises to adjust and change the shape of the suction arrangement, which takes time from the operator's other work. When placing the suction device at the bottom of the mouth, the suction device's holes often get blocked since the mobile part of the mucous membrane is absorbed in to the holes and this leads to a removal or to a strong decrease of the device's absorption capacity. Additionally in such cases, often a loud and very disturbing noise arises when the absorbed mucous membrane vibrates in the suction device's holes.

From the past it is further known to provide plastic tubes with absorptions bodies and place these along each side of the tooth socket or along the row of teeth. Accordingly, some of the above described negative effects and problems connected with saliva absorption can be reduced. Examples of such types of suction devices are described in US 5,071,347 and US 6,309,218.

GB 1 060 288 A relates to a surgical sponge stick comprising a tubular handle of resilient plastics material having a partially flattened portion with perforations through its wall at one end and porous absorbent material forming a surgical sponge mounted on the partially flattened portion covering the perforations.

US 2013/203012 A1 relates to a siphoning device suited for automatically removing fluid/ blood from a surgical site, having a length of sterile flexible tubing that is configured into a loop, the tubing having a first open end, a second open end, and a central portion. At least one or more sets of a plurality of holes punctuating a circumference of the central portion of the loop are provided, which are distributed along a longitudinal direction of the tubing, and sterile absorbent covering encompassing the one or more sets of the plurality of holes, the covering being permeable to bodily fluids.

Anonymous, "J.H. Orsing AB", 18 February 2015, (XP055801872), URL:https:// web.archive.org/web/20150218042400/http://orsing.se/products/saliva-ejectors/ hygoformic/ discloses a dental suction arrangement for the removal of saliva and other fluids, the arrangement having a suction line having a prefabricated shape and size, made from an elastically deformable material. The arrangement does not comprise an absorption body and does not comprise a tongue holder.

In order to efficiently absorb saliva and water from the oral cavity using such an absorption body it is important that the absorption body has good connection/coverage with the area where the saliva is produced and accumulated. The absorption body of the above mentioned known suction arrangement is cylindrical. This results in an incomplete connection with the respective area. Certainly some of the absorption bodies have some elasticity which allows the absorption body to compress so that the area of the connection points can be expanded, however only limited. This leads to an unsatisfactory absorption, why the advantages in working only with absorption tubes are limited.

These devices are very static and the absorbing bodies are placed along the row of the teeth in a fixed device, absorption will only take place when the saliva/fluid reaches a certain level and get in contact with the bodies. The area between the bodies are kept dry but the all the remaining fluid and saliva will reach the throat of the patient as if there hasn't been any suction device at all.

Another disadvantage of the - under known technic - cylinder-shaped absorption bodies is that they are placed along the tooth socket. When saliva accumulates in the oral cavity and rises to a level so that it tends to flow over in the patient's throat then the patient reflexively will swallow, and also this swallowing movement will increase the level of saliva and water.

Thus there exists a need of a suction arrangement which removes the saliva and water where it accumulates, i.e. at the bottom of the mouth, so that the operating area is kept dry and water-free and the patient needs to swallow are minimized, and especially not causes discomfort for the patient when the suction arrangement comes in contact with the inside of the lower jaw.

### Summary of the invention

One object of the present invention is to remove the problems associated with the prior known technic in the field and thus provide a more effective absorption of saliva and other fluids than previously offered.

The invention is defined by the appended independent claim 1. Preferred embodiments are defined in the dependent claims.

According to the invention, the suction arrangement is made of elastically deformable material in order to adapt to the anatomical shape of the bottom of the mouth.

Through the elastically deformable material, the main part of the outer surface of the suction arrangement will be connected with areas at the bottom of the mouth from where the fluid will be absorbed. This enables a more effectively and completely absorption.

Also, it is important that the suction line with the absorption body extends not only to the lower part of the lower jaw but that it extends along the lower part of the lower jaw. By this, the absorption body is sort of clamped between the tongue and the lower jaw, such that it will not unintentionally disengage from this place.

This is especially important when the patient swallows as with the prior art devices, due to intense movements of the oral cavity, the suction tube usually then got lost.

The suction arrangement, which comprises elastically deformable material, adapts to the bottom of the mouth and covers the area without causing any discomfort for the patient and at the same time preventing the saliva to come in contact with the teeth, and further the device is retained at the bottom of, the mouth since it is partially placed under the tongue. The suction line may be deformed as desired to be adapted to the best position. It may extend over the molars or incisors to then extend close to and along the lower jaw in the distal direction. Preferably, it reaches the lower part of the lower jaw, i.e. a level level below main tongue extension close to the canine, and then extends under tongue along the premolars and molars. The suction line is preferably made of plastic material which is easy and plastically deformable. The absorption body may be fixed the suction part of the suction line in any suitable manner, such as by glueing or by friction. The suction arrangement is suitable both for to be applied to the left or the right lower jaw.

The absorption body within the suction arrangement also works as a pump when it is compressed due the movement when the patient swallows and the subsequently expansion.

In order to ensure the desired and beneficial pump action, it it essential for the elastic absorption body to have a compressibility to half of its original volume by standard tongue force. The compressibility may be considerable higher, i.e. up to 85 percent. Thus, the material of the absorption body may be soft open foam with a low elasticity coefficient, or a soft flocking, or a silky mesh covering the inner absorption body.

The absorption body also eliminates the need to interrupt e.g. the ongoing dental cleaning treatment for adjustment of the suction device when it causes painful pressure to the patient's inside of the lower jaw which is very pain sensitive.

Due to the fact that the suction arrangement adapts itself to the anatomical shape of the bottom of the mouth and also is elastic, it automatically very efficiently adapts to the exact anatomical shape in a way which is not possible with a simple cylindrical absorption body under known technique. Also the dental treatment will be more silent since absorption body acts as noise shield, dampening the suction noise emitted by suction holes. The created anatomical form effectively allow the reception of saliva leaving both Glandula Sublingualis and Glandula Submandubularis, and the device builds a barrier between the teeth and the saliva glands.

In one embodiment of the invention, the absorption body shows, in a cross sectional view, perpendicular to the length extension of the absorption body, a non-circular contour.

In another embodiment, the absorption body shows, in a in a cross sectional view perpendicular to the absorption body's length extension, a contour with a base and a top, where the mentioned base is broader than the mentioned top. This shape facilitates the placement and retention under a part of the tongue. In another embodiment, the absorption body has a part, which, in a cross sectional view perpendicular to the absorption body's length extension, shows a contour with at least one concave part. With the concave contour part the shape of the absorption body is adapted in a natural way in order to create space for the tongue. This will avoid concentration of pressure at the tongue and at the same time the absorption body effectively reaches areas, over as well as under the tongue, where the absorption is most needed.

Preferably, the cross section of the absorption body is larger below the area of the waist which is created by the tongue and slightly smaller above this waist. The suction part of the suction tube' is preferably arranged at the lower, broader part. In one embodiment, the suction tube is made of elastically deformable and adjustable material in order to obtain an adaption to the anatomical shape of the bottom of the mouth and placement position. This embodiment includes a variety of application possibilities. The suction device and its related suction tube or suction line and absorption body especially can adapt itself to different sizes of the bottom of the mouth, but also since the absorption body is so long it has the possibility to extend along the alveolar area or gingival area of the jaw at the incisors and canines as well as at premolars and molars, and due to the cross sectional shape it adapts to the different parts.

In a further embodiment of the invention, the suction device includes an extension part which, when placed in the oral cavity, extends backwards towards the throat, whereby the extension part preferably has a reduced cross-sectional area which decreases in the direction of the throat. The extension part reaches a bit down in the throat which helps reducing the accumulation of fluid in the throat, which otherwise may cause discomfort and disturb the dental treatment due to swallowing reflexes. The extension part generally entails a lower fluid level.

In a further embodiment, the absorption body is adjustable in its length. A long absorption body will be prefabricated, and it will be shortened but cutting it. Alternatively, the absorption body may be fixed by friction on the suction part of the suction line, and be manually extended and shifted according to the needs. Further alternatively, the suction part may be provided with a punch pattern, allowing its extension together with the absorption body.

The hole member comprises multiple holes. This facilitates the transport of fluid from the outer surface of the absorption body to the absorption part or suction part since the transport routes become shorter. The multiple holes are distributed along the absorption part as well at its circumference.

In a further embodiment, the holes are of different size and/or shape. The hole size and/or shape or both can thus be adjusted to local variations in relation to the need of absorption at the different points of the absorption body. The need of absorption may vary depending on where the saliva is produced, on locations where saliva tends to accumulate, on locations where other fluids accumulate and on variations in the absorption body's deformation. Also from a flow technical point of view it is favorable to be able to have different sized holes depending on their distance to the connection of the absorptions part to the rest of the suction device.

In a further embodiment, the suction device includes a secondary absorption body to be placed at the Glandula Parotis excretory duct, where the secondary absorption body is in fluid connection with the absorption body.

A substantial part of saliva is produced by the Glandula Parotis excretory duct, i.e. a salivary gland located in the back upper cheek area. By arranging an absorption body even in this area, it is possible to absorb the saliva already at the source where it is produced, and it is not necessary to take care of it when it accumulates into the bottom of the oral cavity. Additionally, it also prevents the saliva from the Glandula Parotis to reach the teeth in the upper jaw's side areas, which is beneficial and comfortable to the patient This results in an increased efficiency of the suction device.

In a further embodiment, the secondary absorption body and the absorption body are connected with each other via a connecting line which can be called secondary suction line. It would also be possible to achieve the transport by capillary action through an extension of the absorption body to the secondary absorption body. However, the flow becomes more efficient in case if performed through a line between the both bodies.

In a further embodiment, the suction device includes multiple absorption bodies and the suction tube includes additional lines, whereby each additional line is connected with a suction part in the respective absorption body.

In this embodiment, the secondary suction line may be formed such that a spring force is exerted between secondary absorption body and the primary absorption body. By this, both are kept in place, and the primary absorption body is pushed down to be closer to Glandula Lingualis.

This embodiment allows for a simultaneously fluid removal from several locations in the oral cavity with just one suction device. The user can decide freely whether absorption bodies should be placed on two or several different points on the right side between the tongue and the row of teeth, on the right side between the row of teeth and cheek, correspondingly on the left side, between the row of teeth and lip, at the Glandula Parotis or on other points in the oral cavity.

In a further embodiment, the respective suction part is detachably connected with the rest of the suction line.

This allows an easy replacement of absorption bodies in case of some reason the absorption capacity would decline. This also allows that one and the same suction tube can be connected to different types of absorption bodies.

In a further embodiment, the suction device includes a suction line and a set of multiple absorption bodies in different embodiments in respect of the anatomical shape and/or size.

With such a set a high flexibility is achieved, since a single suction line can be connected to any type of absorption bodies depending on the current situation. Alternatively, it allows having a set of one absorption body-type in different sizes to choose between for an optimal fitting depending on the patient's jaw size or other patient dependent variables.

In a further embodiment, the suction device includes a protection shield which may be a tongue protection in order to block the tongue. The tongue protection aims to protect the tongue from rotating instruments during the dental treatment such as cleaning treatment, and also to prevent the tongue from touching the tooth or teeth in critical areas which may cause a contamination with saliva when performing different filling and/or cementation of prosthetic constructions.

In one embodiment, the tongue protection is arranged at an extension of the suction line, and in another embodiment, the tongue protection is arranged at one sidearm of the mentioned suction line.

In a further embodiment, the tongue protection is made of a material which is elastically deformable. With this embodiment, the advantage is obtained that the tongue protection can be bent in order to effectively separate the tongue from the jaw area, while also allowing the assisting personal to obtain full access and insight in any critical area for required interventions and complementary removal of water and saliva.

In one embodiment, the tongue protection is made of a material which can be broken off in order to adjust the fitting and placement in the oral cavity and also in order to enable an increased access and insight, while the ability to protect the tongue is retained.

In a further embodiment, the tongue protection is arranged adjustably to the suction tube. This is an additional opportunity to perform specific adjustments depending on the condition of the patient.

In a further embodiment, the tongue protection has an absorption capacity. This absorption capacity can either be provided in case the tongue protection is made of absorbable material, or covered any absorption material or in case the tongue protection is equipped with a - secondary - suction line which is connected with the suction device and its absorption body.

In one embodiment, the respective tongue protection and suction part are detachable connected with the rest of the suction tube.

The secondary suction line between the tongue protection and the absorption body may be such that it is bias to separate the tongue from the lower part of the lower jaw. By this, the absorption body is pressed downward towards the Glandulae under the tongue. The secondary suction line advantageously is bent by about a little less than 180 degree.

In another embodiment of the invention, the tongue holder may be provided with a grip, like a small lateral extension. By this, any operator like a dentist may pull the tongue holder sidewards or in any suitable direction.

In another embodiment of the invention, the tongue holder is turnable and/or attached via a plastic hinge to the secondary suction line.

The tongue holder has a structured surface. This is for the tongue - which tends to like some activity - to play with it.

In another embodiment of the invention, the tongue holder is made from thermosetting material.

The operator then may heat it before use, and shape it to any desirable form. After re-cooling it will maintain the selected form. This process may be repeated frequently if desired.

The tongue holder advantageously may be made from orange acrylic material to form a light curing shield.

The suction line may be made of any suitable material such as special plastic material, The material may be selected to have a memory effect, and/or may be plastically and/or elastically deformable.

In one embodiment, the suction device includes a suction tube or suction line and a set of multiple absorption bodies in different executions in respect of the anatomical shape and/or size, and a set of multiple tongue protections in different executions in respect of the anatomical shape and/or size.

In a very advantageous embodiment of the invention, the absorption body is covered be a silky mesh, such as known from jewelry boxes. Such a web or mesh has a surface with a low friction coefficient such such the absorption body may be inserted easily to its desired place. It has a very pleasant and comfortable feeling for the patient. Yet its permeability is sufficient to have the desired suction force retained therethrough.

Preferably, the silky mesh is very thin and thus very flexible. Also, the absorption body is soft and flexible and has an elastic character. If it is made from foam or any other suitable material, a hardness gradient may be used such that it is harder close to the suction part and softer close to its outer periphery.

Such a hardness gradient automatically is provided if the absorption body is made of a flocking. Any desired shape and distribution is possible. Flocking has a very good capillary effect.

In another embodiment, the suction arrangement is provided with a moisture sensor, intended to give a feedback to the vacuum pump and to reduce the pump action if the area under the tongue is dry. This will also reduce the vacuum pump noise.

The inventive suction arrangement is also very suitable for unteethed patients. Because of the spring action both to the absorption body between the tongue and the bottom of the mouth, and of the suction line between the chin and the lower part of the lower jaw, the invention works great independently of whether the jaws are fully teethed, partially teethed or unteethed.

Other purposes, features and facilities of the invention will be shown in the following detailed description, in the patent claims, as well as in the drawings. It ought to be understood that additional advantageous embodiments may arise via different possible combination of the features from the described embodiments and with each possible combination of these features described in the examples following presentation.

Generally, the terms in the requirements should be interpreted in accordance with their normal meaning in the technical field, unless explicitly stated otherwise. All references to "a / an / the / [a suction arrangement, a suction tube, an absorption, etc.] " should be interpreted openly as a reference, to the existence of at least one mentioned suction device, suction tube, absorption body etc. unless explicitly stated otherwise.

### Brief description of the drawings attached

These and other embodiments of the invention will now be described in more detail with reference to the attached drawings, which show embodiments of the invention.
Fig.1 is a side view of a suction device or suction arrangement
Fig. 2 is a longitudinal section through the suction device in Fig. 1.
Fig. 3 is a sectional view along line III-III in Fig. 2.
Fig. 4 is a sectional view corresponding the one in Fig. 2 illustrating the fluid flow.
Fig. 5 is a sectional view corresponding the one in Fig. 3 illustrating the fluid flow.
Fig. 6 is a top view of the suction device in Fig. 1 placed at the bottom of the mouth between the lower jaw's alveolar area and the tongue.
Fig. 7 is a sectional view along line VII-VII in Fig. 6.
Fig. 8 is a top view of the suction device in Fig. 1 placed at the bottom of the mouth in a displaced position in relation to Fig. 6.
Fig. 9 illustrates a detail of the suction device according to one embodiment of the invention.
Fig. 10-14 illustrates a sectional view through a detail of the suction device according to five different embodiments of the invention.
Fig. 15 is a side view of a further embodiment of a suction device according to the invention.
Fig. 16 is a top view of the suction device in fig. 15.
Fig. 17 is a side view of an embodiment of a suction device according to the invention.
Fig. 18 illustrates a detail of the embodiment in Fig. 17.
Fig. 19 shows a sectional view of another embodiment of the suction arrangement of the present invention, with a modified absorption body.
Fig. 20 another embodiment of the suction arrangement according to the invention.
Fig. 21 still another suction arrangement.

### Description of embodiments

The invention will now be described in more detail with references to the attached drawings.

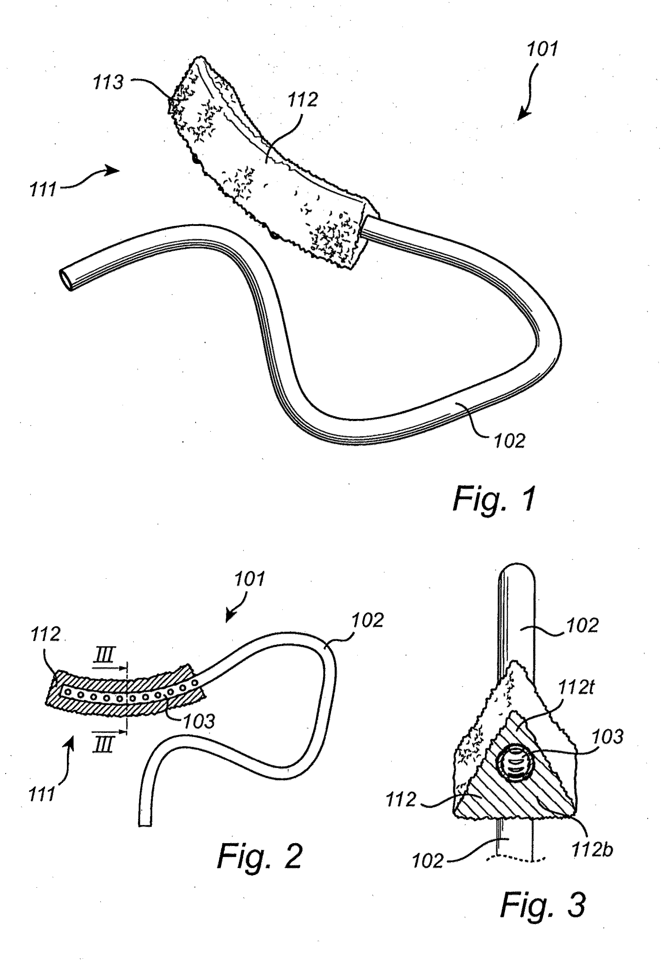

In Fig. 1 to 8, a first suction arrangement is illustrated. As shown in Fig. 1, the suction device has a suction tube or suction line 101 which can be connected to a vacuum source in order to create a negative pressure inside the suction tube. The suction tube 101 has an exposed part 102 which is visible. The suction tube 101 extends into the absorption body 111 of the suction device with a suction part 103 embedded in the absorption body (not visible in Fig. 1). The absorption body 111 consists of a main part 112, and optionally of an extension part 113. The main part 112 is arranged to be placed at the user's bottom of the mouth between the lower part of the lower jaw and the tongue.

The extension part 113 is arranged to extend backward from the main part 112 towards the patient's throat. The exposed part 102 of the suction tube is conventionally bent to easily be fixed around the patient's chin. The suction device is shown in Fig. 2 in a sectional view of its length extension whereby suction part 103 of the suction tube 101 is visible. In this example, exposed part 102 of the suction tube is shown. The suction part 103 may alternatively be jointed to the juncture between the suction part 103 and the exposed part 102. The suction part 103 is equipped with multiple holes 104 which connect the tubes inner part with the covering absorption body. In this example, the absorptions body is smoothly arch-shaped, but since the suction device is made of elastic deformable material the device can adapt other shapes in order to fit the patient's oral cavity and the bottom of the mouth in particular.

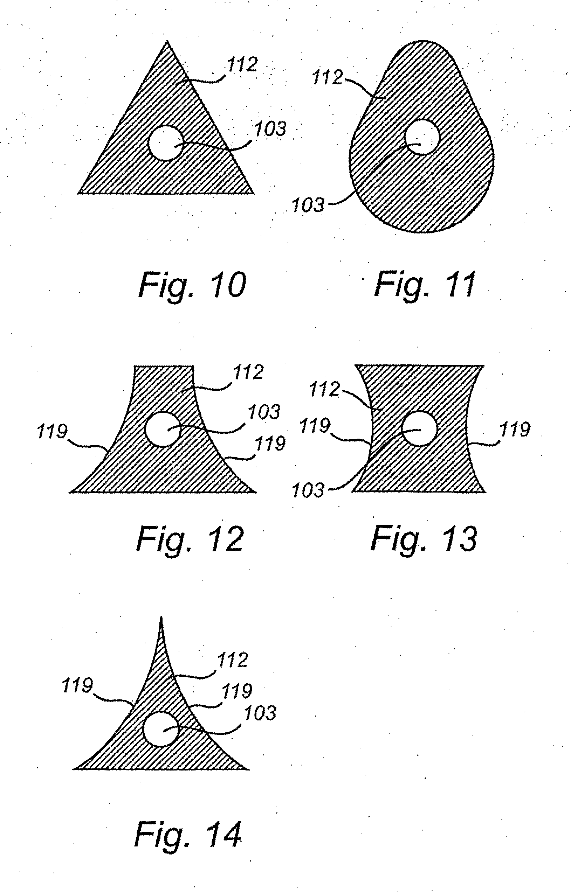

Fig. 3, which is a sectional view along line III-III in Fig. 2, shows that the absorption body in the lateral direction has a non-circular outer contour with a base 112b and a top 112t, whereby the base 112b is broader than the top 112t, and shows a triangular-like cross section. The suction part 103 of the suction tube 101 is centrally located in the absorption body. Alternatively to the illustration, this part can be located in other positions in the absorption body, e.g. in an asymmetric placement. Also, the outer contour may be different than the exemplified. Examples of different contour are shown in Fig. 10 to 14.

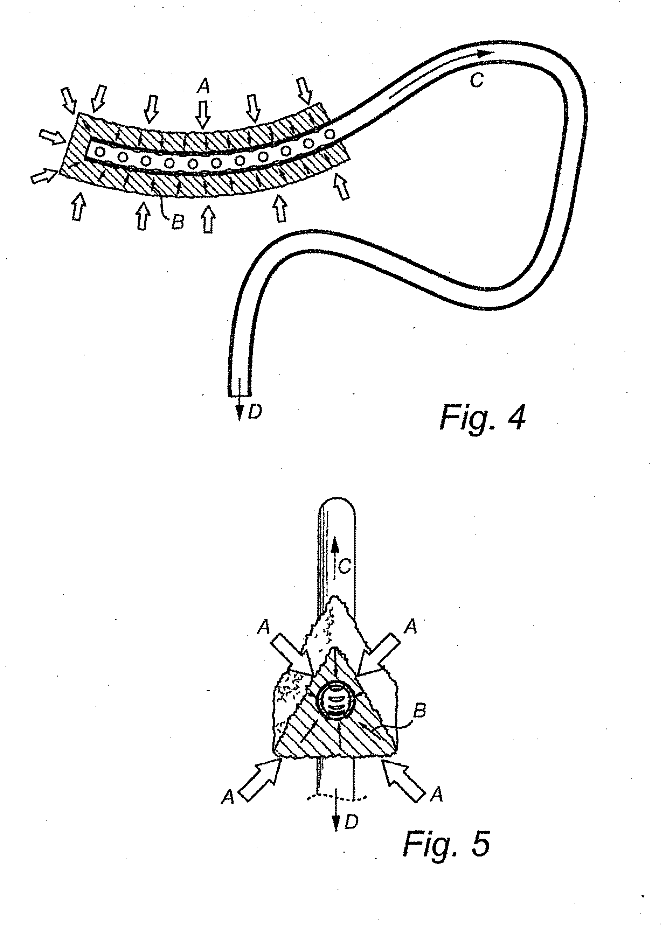

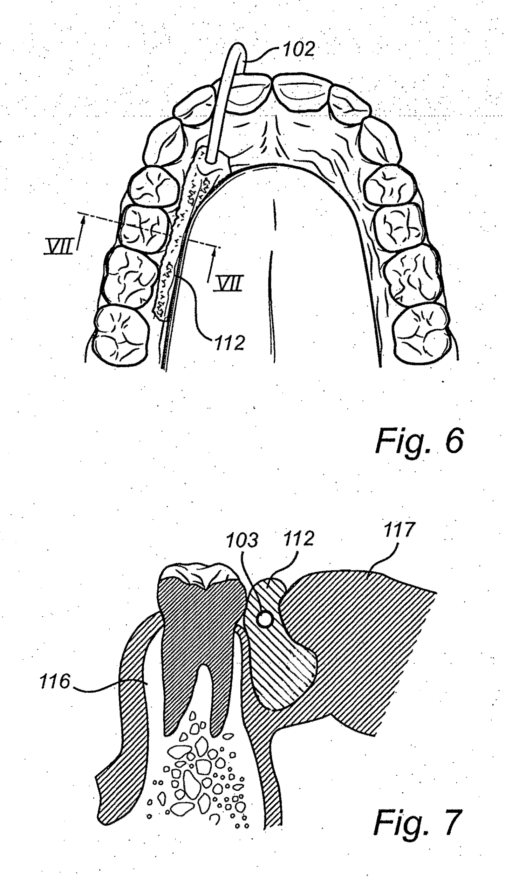

The Figures 4 and 5 schematically show the fluid flow through the suction device. Saliva and water enter the absorption body 111 from mucous membranes and from other places in the oral cavity, where the fluid accumulates, as indicated by arrows A. The suction tube 101 is connected to vacuum whereby under pressure is generated inside its suction part 103. By capillary action the fluid entering the absorption body 111 is absorbed in the direction of the suction part 103, as indicated by arrows B. Through the vacuum the fluid is absorbed by the suction tube according to the arrow C, and is removed according to arrow D. Fig. 6 and 7 illustrate the suction device in place at the bottom of the mouth between the lower part of the lower jaw 116 and the tongue 117.

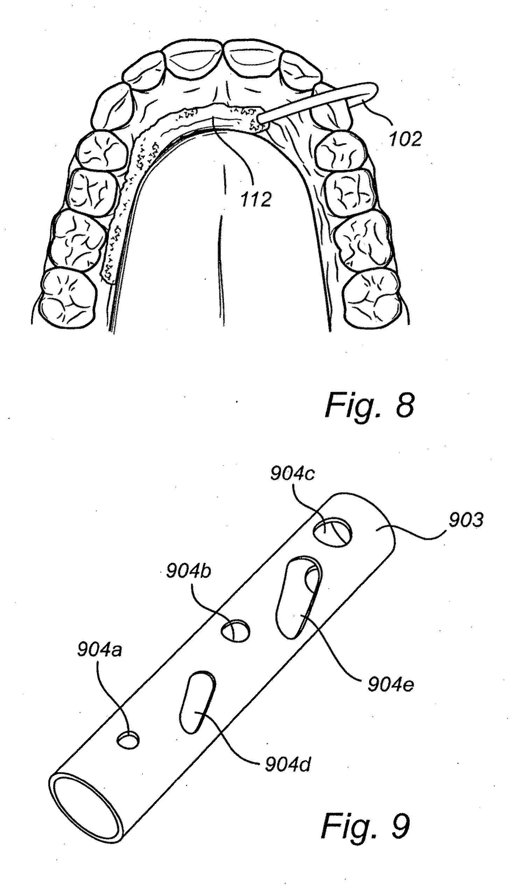

Fig. 8 illustrates the suction device placed in a slightly displaced position in relation to fig. 6. This positioning can be beneficial in filling and cementation of prosthetic constructions in the front part of the patient's mouth. Since the suction device is made of elastic deformable and adjustable material, the suction device can be bent and be placed at the bottom of the mouth beside the lower part of the lower jaw by the incisors and extend back toward the lower part of the lower jaw by the premolars and molars on the right and/or left side.

The suction tube 101 is preferably made of extruded plastic and if needed also includes an integrated wire that allows the operator to reshape the tube if necessary. The suction tube has prefabricated shape and size that allows it to be easily placed at the bottom of the mouth and at the same time to cover the lower part of the lower jaw for a secure fixation.

The suction tube may have a round shape or an oval shape which is even more easy to bend. Also, the wire may be flat, having a softer spring characteristic in its flat direction. Both may be adapted to the user's needs.

The suction part 903 of the suction tube can, as illustrated in Fig. 9, be provided with holes of different size and shape. The holes 904a, 904b, 904c are circular with different diameter where the holes 904a closest to the exposed part of the suction tube or line are portion are the smallest. The holes 9d and 9e are elongated.

The absorption body 111 is made of suitably elastically deformable material which enables fluid transport through the body, preferably foam plastic with open cells. It is also possible to use other soft materials that have capillary action like for example, cotton fibers, various textile fibers, synthetic fibers, mull, lint or gauze, preferably covered by a mesh, and the like.

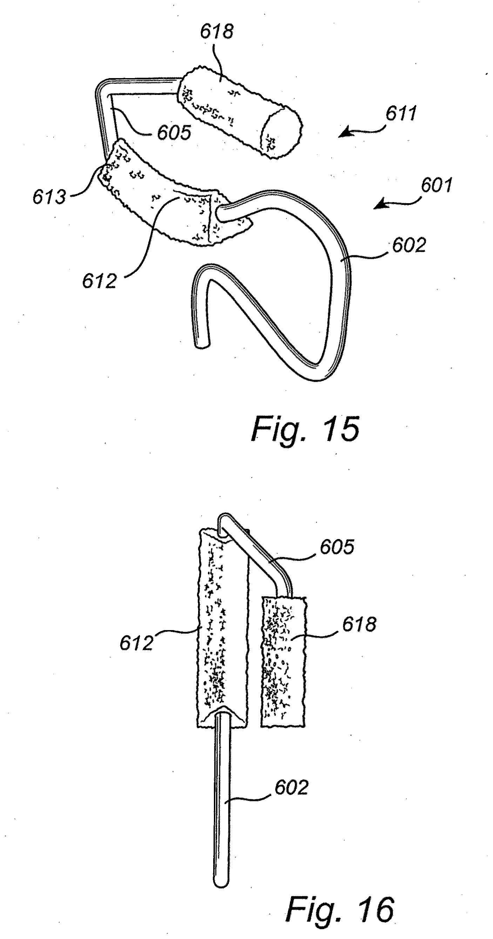

In the embodiment example illustrated in Fig 15 and 16, the suction device includes a ` secondary absorption body 618 beyond the main absorption body 612, 613. The secondary absorption body 618 is arranged to be placed at the Glandula Parotis excretory duct and is connected with the main absorption body via a connecting line 605. The main absorption body is arranged for an application as illustrated in Fig. 6. Via the secondary absorption body 618, saliva from the Glandula Parotis is directly absorbed where it is generated and via the main absorption body transferred to the suction tube 602. The connecting line 605 can advantageously be used as an extension of the suction part of the suction tube, but an arrangement on an additional line of the exposed part 102 of the suction tube is also possible or an arrangement as an additional line to the suction part 103 before the transition to an extension part.

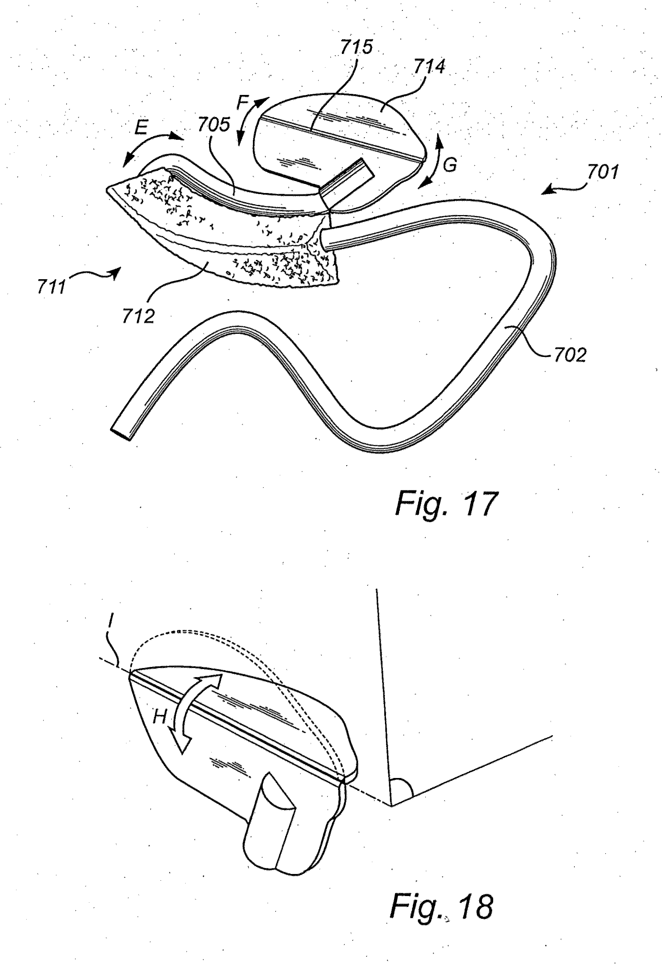

In the embodiment example illustrated in Fig. 17, the suction device comprises a tongue protection 714. The tongue protection aims to block the tongue and so protect the tongue from rotating instruments during the dental treatment and also to prevent the tongue from touching the tooth or teeth in any critical area which may cause a contamination with saliva when performing different filling and/or cementation of prosthetic constructions. Fig. 17 illustrates an embodiment, where the tongue protection 714 is arranged in an extension 705 of the suction tube 702, but in another (not illustrated) embodiment, the tongue protection may be is arranged on one sidearm of the exposed suction tube 702. In a further (not illustrated) embodiment, the suction device includes two tongue protections, one arranged as illustrated in Fig. 17, and one arranged on one sidearm of the exposed suction tube 702. The tongue protection can be made of elastically deformable material. Thus, the tongue protection can be bent, as illustrated with arrow H in Fig. 18, along line I, and therefore effectively separate the tongue from the operation, while also allowing the assisting personal to obtain full access and insight for required interventions and complementary removal of water and saliva.

In one embodiment, the tongue protection is made of a material which can be broken off, e.g. along line 715, in order to adjust the fitting and placement in the oral cavity and also in order to enable an increased access and insight while the ability to protect the tongue during treatment is retained.

Furthermore, the tongue protection may be arranged in relation to the suction tube's 702 suction part 703 and the absorption body 712, as illustrated by arrows E, F and G in Fig. 17.

In a further (not illustrated) embodiment, the tongue protection has an absorption capacity. This absorption capacity can either be provided in case the tongue protection is made of absorbable material or when the tongue protection is equipped with a suction tube which is connected with the suction device and its absorption body.

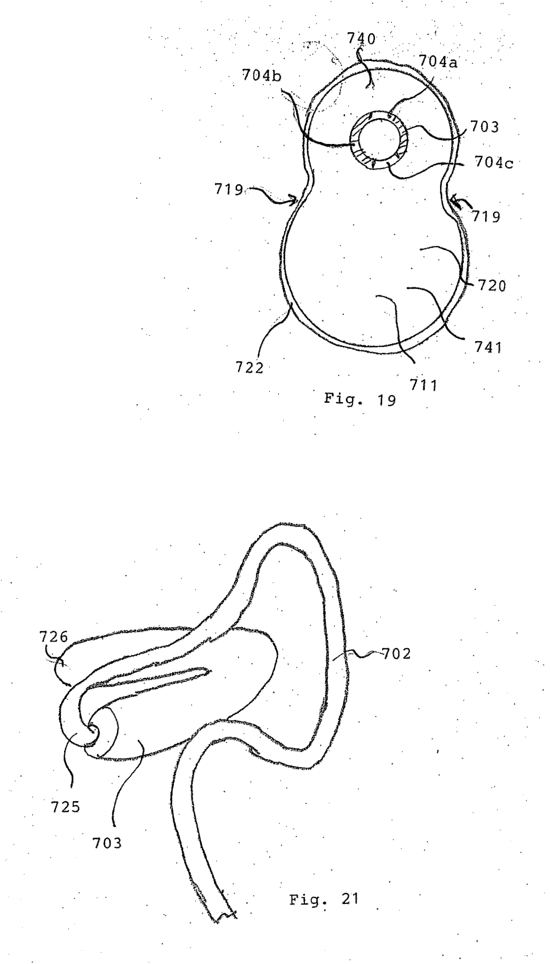

Fig. 19 shows another embodiment of tthe invention. According to this embodiment, the absorption body 711 is formed such as it is noncircular, with a base and a top, whereby the base 741 is broader than the top 740. The general form corresponds to an egg, however with a waist such that having concave parts 719 separating the top portion 740 and the base portion 741.

According to this embodiment, a huge suction buffer is provided which is intended to at least partially fill-out the space under the patient's tongue. Also, with such a huge buffer, the desired pump action by the force exerted by the tongue is improved.

The absorbtion body 711 is made by open-porous foam with a high elasticity, i. e. a soft open foam. This foam, according to the shown embodiment, is covered by a silky mesh which is also open-porous, to let the fluid pass, and is pleasent and comfortable to the patient. Alternatively, it is closed-porous to block fluids.

Fig. 21 shows another embodiment of the inventive dental suction arrangement.

A suction part 703 passes through the top portion 740. It has a plurality of suction holes 704a, 704b and 704c which are distributed uneven and with different diameters and forms all over the suction part 703.

Preferably, the suction part 703 extends in the centre of the top portion 740. In the different embodiment it may extend excentrically, in order to increase the suction power at one side of the absorption body 711.

According to this embodiment, a tongue holder 714 is arranged at the end of a second suction line 705. The absorption body 711 is arranged between the second suction line 705 and first suction line 702. It comprises a suction part 703 with a plurality of suction holes 704.

As may be taken from the arrows C, D and E, all parts of this embodiment may be easily adjusted and bended according to the needs. Generally, it is prefered to have the second suction line 705 being bent by about 150 to 180 degree and also the first suction line 702 bent several time:
The idea is to have the absorption body 703 being arranged as low as possible under the patients tongue in the bottom of the mouth, close to glandolar submandibularis and glandouar sublingualis, and the tongue holder 711 extending upwardly from there and pressing the tongue obliquely upwardly and away from the jaw.

The first suction line 702, on the other hand, will cross over the canines or incisors of the patient and leave the mouth, to be attached to a suction source like a vacuum pump.

By such an arrangement, both the absorption body 703 is pressed down, and the tongue holder 711 is pressed upwardly. Thus, it is prefered to have a certain section in the second suction line which should be bendable, in order to adapted to the patients needs.

Another embodiment is shown in Fig. 21. This embodiment comprises a suction line 702 and a so-called "full jaw" absorption body 703. This absorption body is essentially U shaped and comprises a suction part with suction holes even this is not shown in Fig. 21. It is intended to be placed under the tongue and the suction line 702 is of a flexibility such that it may be bended at a hinge area 725, to have this essentially U-shaped absorption body being inserted into the oral cavitiy either such that the suction line 702 is arranged on the left side or on the right side.

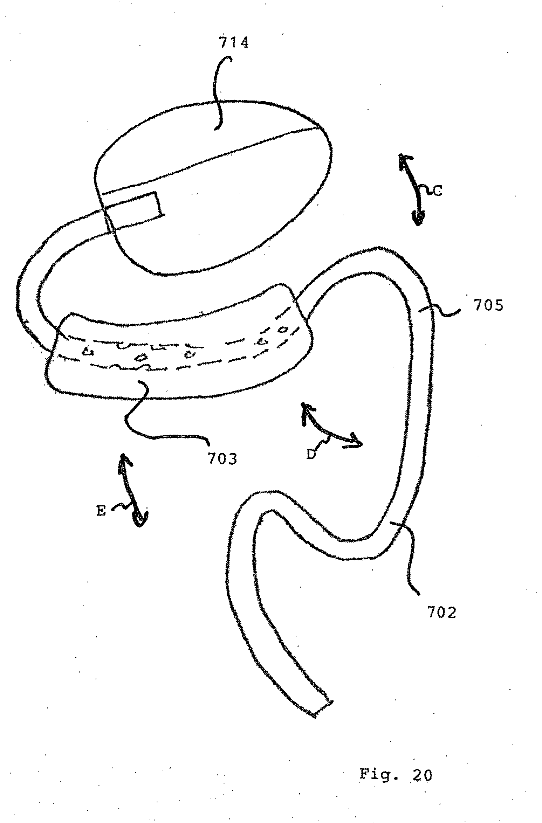

In another embodiment which is not shown, an additional tongue holder is arranged at the end 726 of the absorption body 703. The form thereof may be similar to the form shown in Fig. 20, and a second suction line may be provided between the tongue holder 714 and the end 726.

The invention has been described in relation to the current understanding of which are the most practical and preferred embodiments, but it is recognized that the invention is not limited to the described embodiments; several variations and modifications are possible. The scope of the invention is therefore exclusively defined by the attached patent claims.

## Claims

1. Dental suction arrangement for the removal of saliva and other fluids, where the suction arrangement includes a suction line (101, 602, 702) and an absorption body (111, 611, 711) to be placed for the removal of saliva and other fluids, whereby the suction line (101) includes a suction part (103) which is arranged in the absorption body (111) and which is equipped with hole members (104, 904a-e) communicating with the absorption body, wherein the suction line (101, 602, 702) has a prefabricated form and size for positioning said absorption body (111) at the bottom of a cavity, or close to said bottom, wherein the absorption body (111, 611, 711) is made of elastically deformable and adaptable material in order to adapt it to the desired shape, and said hole members (104, 904a-e) are distributed along the longitudinal extent of said suction part (103) as well as on its periphery, and that the suction part (103) is detachable connected with the rest of the suction line (102) together with its associated absorption body (111) and the suction tube 101 has an exposed part 102 which is visible and bent to be fixed around the patient's chin and said suction line (101,602,702) is configured to extend along an elevated portion upward from said bottom, and at least partially surrounding said portion for secure fixing, and the dental suction arrangement comprises a tongue holder (714) that has a structured surface.

2. Dental suction arrangement according to claim 1, **characterized in that** the absorption body (111, 611, 711), in a cross-sectional view perpendicular to the longitudinal extent of the absorption body, has a non-circular contour and/or has a contour with a base and a top, whereby the mentioned base is broader than the mentioned top, and/or has a contour with at least one concave part (119).

3. Dental suction arrangement according to any of the previous claims, **characterized in that** the absorption body (111, 611, 711) includes an extension part (113, 613) which, preferably when placed in the cavity, extends distal from the suction line (101, 601, 701), whereby the extension part (113, 613) preferably has a reduced cross-sectional area which decreases in the direction of its distal end.

4. Dental suction arrangement according to any of the previous claims, **characterized in that** the absorption body (111, 611, 711) is adjustable in its length, preferably by being ductily deformed, and/or has a base body with a punch pattern allowing said body together with a foam body of the absorption body to be extended.

5. Dental suction arrangement according to any of the previous claims, **characterized in that** the absorption body (111, 611, 711) is surrounded by, or made of, a mesh with a silky look-and- feel, or by a soft flocking, said mesh or flocking having a capillary effect.

6. Dental suction arrangement according to any of the previous claims, **characterized in that** said mentioned multiple holes comprise holes (104, 904a-e) of different size and/or different form.

7. Dental suction arrangement according to any of the previous claims, **characterized in that** it includes a secondary and adjustable suction line (605) extends from the main absorption body (602, 603, 611) for connection with a secondary absorption body (618) and/or that the suction arrangement comprises multiple absorption bodies and that the suction line comprises additional lines, whereby each additional line is connected with a suction part in the respective absorption body

8. Dental suction arrangement according to any of the previous claims, **characterized in that** the suction arrangement comprises a suction line (102) and a set of a multiple absorption bodies (111, 611, 618, 711) in different embodiments in respect of shape and/or size.

9. Dental suction arrangement according to any of the previous claims, **characterized in that** the absorption body (111, 611, 711) and the suction line (101, 601,701) running through it, i.e. the suction part (103), are essentially U-shaped, said suction line (101, 601,701) exiting from the absorption body (111, 611, 711) at an end thereof .

10. Dental suction arrangement according to any of the previous claims, **characterized in that** the dental suction arrangement further includes at least one protection shield for withholding a third part from moving toward any part of the dental suction arrangement.

11. Dental suction arrangement according to claims 10, **characterized in that** at least one of the mentioned protection shields is arranged at an extension of the suction line (102) and/or **in that** at least one of the mentioned protection shields is arranged on a sidearm of the suction line (102). and/or that the respective suction part (103) and protection shield are detachable connected with the rest of the suction line (102).

12. Dental suction arrangement according to any one of the claims 10 to 11, **characterized in that** the suction arrangement comprises a suction line (102) and a set of multiple absorption bodies (111, 611, 711) in different executions in respect of shape and/or size, and a set of multiple protection shields in different embodiments in respect of shape and/or size.

13. Dental suction arrangement according to any of the previous claims, **characterized in that** the protection shield is adjustable and especially bendable and/or has at least one hinge and/or has at least one predetermined breaking point.

14. Dental suction arrangement according to any of the previous claims, **characterized in that** protection shield is mounted on a second suction line (705) distal from said absorption body (71 1), said second suction line extending back along said absorption body (71 1), said second suction line (705) being adjustable for any alignment of the protection shield at user's deliberation.

15. Dental suction arrangement according to any of the previous claims, **characterized in that** protection shield is forms a light cure shield and preferably is made from orange acrylic material.

16. Dental suction arrangement according to any of the previous claims, **characterized in that** protection shield has a flat and elongated form, extending as an extension of said suction line (705) and/or has a protrusion extending laterally from its elongated form.

17. Dental suction arrangement according to any of the previous claims, **characterized in that** said cavity is an oral cavity, said elevated portion is a lower jaw, said protection shield is a tongue protection and/or said secondary absorption body (618) is arranged for being positioned at the parotis gland duct and/or the bottom being the area between the tongue and the bottom of the mouth.

## Patentansprüche

1. Dentale Absauganordnung zum Entfernen von Speichel und anderen Fluiden, wobei die Absauganordnung eine Saugleitung (101, 602, 702) und einen Absorptionskörper (111, 611, 711) zum Platzieren zum Entfernen von Speichel und anderen Fluiden umfasst, wobei die Saugleitung (101) einen Saugteil (103) umfasst, der im Absorptionskörper (111) angeordnet ist und der mit mit dem Absorptionskörper kommunizierenden Lochelementen (104, 904a-e) ausgestattet ist, wobei die Saugleitung (101, 602, 702) eine vorgefertigte Form und Größe zum Positionieren des Absorptionskörpers (111) am Boden eines Hohlraums, oder nahe des Bodens, aufweist, wobei der Absorptionskörper (111, 611, 711) aus elastisch verformbarem und anpassbarem Material hergestellt ist, um ihn an die gewünschte Form anzupassen, und die Lochelemente (104, 904a-e) entlang der Längserstreckung des Saugteils (103) sowie an dessen Umfang verteilt sind, und dass der Saugteil (103) mit dem Rest der Saugleitung (102) zusammen mit seinem zugehörigen Absorptionskörper (111) abnehmbar verbunden ist, und die Saugleitung (101) einen freiliegenden Teil (102) aufweist, der sichtbar ist und gebogen ist, um um das Kinn des Patienten befestigt zu werden, und wobei die Saugleitung (101, 602, 702) ausgestaltet ist, sich entlang eines erhöhten Abschnitts nach oben von dem Boden zu erstrecken, und mindestens teilweise den Abschnitt zur sicheren Befestigung umgibt, und die dentale Absauganordnung einen Zungenhalter (714) umfasst, der eine strukturierte Oberfläche aufweist.

2. Dentale Absauganordnung nach Anspruch 1, **dadurch gekennzeichnet, dass** der Absorptionskörper (111, 611, 711), in einer Querschnittsansicht senkrecht zur Längserstreckung des Absorptionskörpers, eine nicht kreisförmige Kontur aufweist und/oder eine Kontur mit einer Basis und einer Oberseite aufweist, wobei die Basis breiter ist als die Oberseite, und/oder eine Kontur mit mindestens einem konkaven Teil (119) aufweist.

3. Dentale Absauganordnung nach einem der vorhergehenden Ansprüche, **dadurch gekennzeichnet, dass** der Absorptionskörper (111, 611, 711) ein Verlängerungsteil (113, 613) umfasst, das sich, bevorzugt wenn es in dem Hohlraum platziert ist, distal von der Saugleitung (101, 601, 701) erstreckt, wobei das Verlängerungsteil (113, 613) bevorzugt eine reduzierte Querschnittsfläche aufweist, die in Richtung seines distalen Endes abnimmt.

4. Dentale Absauganordnung nach einem der vorhergehenden Ansprüche, **dadurch gekennzeichnet, dass** der Absorptionskörper (111, 611, 711) in seiner Länge einstellbar, bevorzugt duktil verformbar, ist und/oder einen Basiskörper mit einem Stanzmuster aufweist, wodurch ermöglicht wird, dass der Körper zusammen mit einem Schaumstoffkörper des Absorptionskörpers verlängert wird.

5. Dentale Absauganordnung nach einem der vorhergehenden Ansprüche, **dadurch gekennzeichnet, dass** der Absorptionskörper (111, 611, 711) von einem Geflecht mit einer seidigen Optik und Haptik, oder von einer weichen Beflockung umgeben ist oder daraus hergestellt ist, wobei das Geflecht oder die Beflockung einen Kapillareffekt aufweisen.

6. Dentale Absauganordnung nach einem der vorhergehenden Ansprüche, **dadurch gekennzeichnet, dass** die mehreren Löcher Löcher (104, 904a-e) unterschiedlicher Größe und/oder unterschiedlicher Form umfassen.

7. Dentale Absauganordnung nach einem der vorhergehenden Ansprüche, **dadurch gekennzeichnet, dass** sie eine sich vom Hauptabsorptionskörper (602, 603, 611) erstreckende einstellbare Sekundärsaugleitung (605) zur Verbindung mit einem Sekundärabsorptionskörper (618) umfasst und/oder dass die Absauganordnung mehrere Absorptionskörper umfasst und dass die Saugleitung zusätzliche Leitungen umfasst, wodurch jede zusätzliche Leitung mit einem Saugteil im jeweiligen Absorptionskörper verbunden ist.

8. Dentale Absauganordnung nach einem der vorhergehenden Ansprüche, **dadurch gekennzeichnet, dass** die Absauganordnung eine Saugleitung (102) und einen Satz mehrerer Absorptionskörper (111, 611, 618, 711) in unterschiedlichen Ausführungsformen in Bezug auf Form und/oder Größe umfasst.

9. Dentale Absauganordnung nach einem der vorhergehenden Ansprüche, **dadurch gekennzeichnet, dass** der Absorptionskörper (111, 611, 711) und die durch ihn verlaufende Saugleitung (101, 601, 701), d.h. der Saugteil (103), im Wesentlichen U-förmig sind, wobei die Saugleitung (101, 601, 701) den Absorptionskörper (111, 611, 711) an einem Ende davon verlässt.

10. Dentale Absauganordnung nach einem der vorhergehenden Ansprüche, **dadurch gekennzeichnet, dass** die dentale Absauganordnung ferner mindestens einen Schutzschild umfasst, um ein drittes Teil daran zu hindern, sich in Richtung eines beliebigen Teils der dentalen Absauganordnung zu bewegen.

11. Dentale Absauganordnung nach Anspruch 10, **dadurch gekennzeichnet, dass** mindestens einer der Schutzschilder an einer Verlängerung der Saugleitung (102) angeordnet ist und/oder dass mindestens einer der Schutzschilder an einem Seitenarm der Saugleitung (102) angeordnet ist und/oder dass jeweils Saugteil (103) und Schutzschild mit dem Rest der Saugleitung (102) abnehmbar verbunden sind.

12. Dentale Absauganordnung nach einem der Ansprüche 10 bis 11, **dadurch gekennzeichnet, dass** die Absauganordnung eine Saugleitung (102) und einen Satz mehrerer Absorptionskörper (111, 611, 711) in unterschiedlichen Ausführungsformen in Bezug auf Form und/oder Größe umfasst, und einen Satz mehrerer Schutzschilder in unterschiedlichen Ausführungsformen in Bezug auf Form und/oder Größe.

13. Dentale Absauganordnung nach einem der vorhergehenden Ansprüche, **dadurch gekennzeichnet, dass** der Schutzschild einstellbar und besonders biegbar ist und/oder mindestens ein Gelenk aufweist und/oder mindestens eine Sollbruchstelle aufweist.

14. Dentale Absauganordnung nach einem der vorhergehenden Ansprüche, **dadurch gekennzeichnet, dass** der Schutzschild an einer zweiten Saugleitung (705) distal des Absorptionskörpers (711) befestigt ist, wobei sich die zweite Saugleitung zurück entlang des Absorptionskörpers (711) erstreckt, wobei die zweite Saugleitung (705) für eine beliebige Ausrichtung des Schutzschildes nach Wahl des Nutzers einstellbar ist.

15. Dentale Absauganordnung nach einem der vorhergehenden Ansprüche, **dadurch gekennzeichnet, dass** der Schutzschild einen Lichthärtschild bildet und bevorzugt aus orangem Acrylmaterial hergestellt ist.

16. Dentale Absauganordnung nach einem der vorhergehenden Ansprüche, **dadurch gekennzeichnet, dass** der Schutzschild eine flache und längliche Form aufweist, die sich in Verlängerung der Saugleitung (705) erstreckt, und/oder einen Vorsprung aufweist, der sich lateral von seiner länglichen Form erstreckt.

17. Dentale Absauganordnung nach einem der vorhergehenden Ansprüche, **dadurch gekennzeichnet, dass** der Hohlraum eine Mundhöhle ist, der erhöhte Abschnitt ein Unterkiefer ist, der Schutzschild ein Zungenschutz ist und/oder der Sekundärabsorptionskörper (618) so angeordnet ist, dass er am Ohrspeicheldrüsengang positioniert ist, und/oder der Boden der Bereich zwischen der Zunge und dem Boden des Mundes ist.

## Revendications

1. Dispositif d'aspiration dentaire pour l'élimination de la salive et d'autres fluides, où le dispositif d'aspiration comprend une conduite d'aspiration (101, 602, 702) et un corps d'absorption (111, 611, 711) à placer pour l'élimination de la salive et d'autres fluides, où la conduite d'aspiration (101) comprend une partie d'aspiration (103) qui est disposée dans le corps d'absorption (111) et qui est équipée d'éléments d'orifice (104, 904a-e) communiquant avec le corps d'absorption, dans lequel la conduite d'aspiration (101, 602, 702) a une forme et une taille préfabriquées pour positionner ledit corps d'absorption (111) au fond d'une cavité, ou à proximité dudit fond, où le corps d'absorption (111, 611, 711) est constitué d'un matériau élastiquement déformable et adaptable afin de l'adapter à la forme souhaitée, et que lesdits éléments d'orifice (104, 904a-e) sont répartis le long de l'étendue longitudinale de ladite partie d'aspiration (103) ainsi que sur sa périphérie, et que la partie d'aspiration (103) est détachable et reliée au reste de la conduite d'aspiration (102) avec son corps d'absorption associé (111) et que le tube d'aspiration 101 a une partie exposée 102 qui est visible et pliée pour être fixée autour du menton du patient et que ladite conduite d'aspiration (101, 602, 702) est configuré pour s'étendre le long d'une partie élevée vers le haut à partir dudit fond, et entoure au moins partiellement ladite partie pour une fixation sûre, et le dispositif d'aspiration dentaire comprend un porte-langue (714) qui a une surface structurée.

2. Dispositif d'aspiration dentaire selon la revendication 1, **caractérisé en ce que** le corps d'absorption (111, 611, 711), dans une vue en coupe perpendiculaire à l'étendue longitudinale du corps d'absorption, a un contour non circulaire et/ou a un contour avec une base et un sommet, où la base mentionnée est plus large que le sommet mentionné, et/ou a un contour avec au moins une partie concave (119).

3. Dispositif d'aspiration dentaire selon l'une quelconque des revendications précédentes, **caractérisé en ce que** le corps d'absorption (111, 611, 711) comprend une partie d'extension (113, 613) qui, de préférence lorsqu'elle est placée dans la cavité, s'étend à distance de la conduite d'aspiration (101, 601, 701), où la partie d'extension (113, 613) a de préférence une surface de section réduite qui diminue en direction de son extrémité distale.

4. Dispositif d'aspiration dentaire selon l'une quelconque des revendications précédentes, **caractérisé en ce que** le corps d'absorption (111, 611, 711) est réglable dans sa longueur, de préférence par déformation ductile, et/ou a un corps de base avec un motif de poinçonnage permettant à ce corps ainsi qu'à un corps en mousse du corps d'absorption d'être étendus.

5. Dispositif d'aspiration dentaire selon l'une quelconque des revendications précédentes, **caractérisé en ce que** le corps d'absorption (111, 611, 711) est entouré ou constitué d'une maille à l'aspect et au toucher soyeux, ou d'un flocage doux, ladite maille ou ledit flocage ayant un effet capillaire.

6. Dispositif d'aspiration dentaire selon l'une quelconque des revendications précédentes, **caractérisé en ce que** lesdits orifices multiples mentionnés comportent des orifices (104, 904a-e) de taille et/ou de forme différentes.

7. Dispositif d'aspiration dentaire selon l'une quelconque des revendications précédentes, **caractérisé en ce qu'**il comprend une conduite d'aspiration secondaire et réglable (605) qui s'étend à partir du corps d'absorption principal (602, 603, 611) pour être raccordée à un corps d'absorption secondaire (618) et/ou que le dispositif d'aspiration comprend plusieurs corps d'absorption et que la conduite d'aspiration comprend des conduites supplémentaires, où chaque conduite supplémentaire est raccordée à une partie d'aspiration dans le corps d'absorption respectif.

8. Dispositif d'aspiration dentaire selon l'une quelconque des revendications précédentes, **caractérisé en ce que** le dispositif d'aspiration comprend une conduite d'aspiration (102) et un ensemble de corps d'absorption (111, 611, 618, 711) multiples dans des réalisations différentes en ce qui concerne la forme et/ou la taille.

9. Dispositif d'aspiration dentaire selon l'une quelconque des revendications précédentes, **caractérisé en ce que** le corps d'absorption (111, 611, 711) et la conduite d'aspiration (101, 601, 701) qui le traverse, c'est-à-dire la partie d'aspiration (103), sont essentiellement en forme de U, ladite conduite d'aspiration (101, 601, 701) sortant du corps d'absorption (111, 611, 711) à l'une de ses extrémités.

10. Dispositif d'aspiration dentaire selon l'une quelconque des revendications précédentes, **caractérisé en ce que** le dispositif d'aspiration dentaire comprend en outre au moins un bouclier de protection pour empêcher une troisième partie de se déplacer vers une partie quelconque du dispositif d'aspiration dentaire.

11. Dispositif d'aspiration dentaire selon la revendication 10, **caractérisé en ce qu'**au moins un des boucliers de protection mentionnés est disposé dans le prolongement de la conduite d'aspiration (102) et/ou **en ce qu'**au moins un des boucliers de protection mentionnés est disposé sur un bras latéral de la conduite d'aspiration (102), et/ou **en ce que** la partie d'aspiration (103) et le bouclier de protection respectifs sont raccordés de manière amovible au reste de la conduite d'aspiration (102).

12. Dispositif d'aspiration dentaire selon l'une quelconque des revendications 10 à 11, **caractérisé en ce que** le dispositif d'aspiration comprend une conduite d'aspiration (102) et un ensemble de corps d'absorption multiples (111, 611, 711) dans différentes exécutions en ce qui concerne la forme et/ou la taille, et un ensemble de multiples boucliers de protection dans différents modes de réalisation en ce qui concerne la forme et/ou la taille.

13. Dispositif d'aspiration dentaire selon l'une quelconque des revendications précédentes, **caractérisé en ce que** le bouclier de protection est réglable et particulièrement pliable et/ou comporte au moins une charnière et/ou présente au moins un point de rupture prédéterminé.

14. Dispositif d'aspiration dentaire selon l'une quelconque des revendications précédentes, **caractérisé en ce que** le bouclier de protection est monté sur une seconde conduite d'aspiration (705) distale dudit corps d'absorption (711), ladite seconde conduite d'aspiration s'étendant vers l'arrière le long dudit corps d'absorption (711), ladite seconde conduite d'aspiration (705) étant réglable pour tout alignement du bouclier de protection au gré de l'utilisateur.

15. Dispositif d'aspiration dentaire selon l'une quelconque des revendications précédentes, **caractérisé en ce que** le bouclier de protection forme un bouclier photopolymérisable et est de préférence fabriqué à partir d'un matériau acrylique orange.

16. Dispositif d'aspiration dentaire selon l'une quelconque des revendications précédentes, **caractérisé en ce que** le bouclier de protection a une forme plate et allongée, s'étendant comme un prolongement de ladite conduite d'aspiration (705) et/ou présente une saillie s'étendant latéralement à partir de sa forme allongée.

17. Dispositif d'aspiration dentaire selon l'une quelconque des revendications précédentes, **caractérisé en ce que** ladite cavité est une cavité buccale, ladite partie surélevée est une mâchoire inférieure, ledit bouclier de protection est une protection de la langue et/ou ledit corps d'absorption secondaire (618) est agencé pour être positionné au niveau du canal de la glande parotique et/ou le fond étant la zone située entre la langue et le bas de la bouche.
